(19) Europäisches Patentamt
European Patent Office
Office européen des brevets

(11) EP 1 264 814 A1

(12) EUROPEAN PATENT APPLICATION

(43) Date of publication:
11.12.2002 Bulletin 2002/50

(51) Int Cl.7: **C07B 57/00**, C07C 307/02

(21) Application number: **01202251.3**

(22) Date of filing: **08.06.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Avantium International B.V.**
**1014 BV Amsterdam (NL)**

(72) Inventors:
- **Smith, Alan Arthur**
**1014 BV Amsterdam (NL)**
- **Damen, Franciscus Wilhelmus Petrus**
**1014 BV Amsterdam (NL)**
- **Kuster, George Johannes Theodorus**
**1014 BV Amsterdam (NL)**

(74) Representative: **van der Kloet-Dorleijn, G.W.F.**
**Exter Polak & Charlouis B.V.**
**P.O. Box 3241**
**2280 GE Rijswijk (NL)**

(54) **Resolving agents based on amines**

(57) The invention relates to a resolving agent consisting of a chiral sulphamic acid derivative of the formula $(R_1,R_2)N-SO_3H$, wherein $R_1$ and $R_2$, being the same or different, represent a hydrogen atom, an optionally substituted, linear, branched or cyclic alkyl or heteroalkyl group, or an optionally substituted aromatic or heteroaromatic group, provided that at least one of the $R_1$ and $R_2$ groups is chiral.

The invention also relates to a process for the resolution of a mixture of enantiomers using said resolving agent and to a process for the preparation of a chiral sulphamic acid derivative wherein an amine of the formula $(R_1,R_2)N-H$, wherein $R_1$ and $R_2$ are as defined above, is reacted with a suitable reagent to obtain said chiral sulphamic acid.

EP 1 264 814 A1

## Description

The invention relates to a resolving agent for resolving a mixture of enantiomers.

More specifically, the invention relates to a resolving agent consisting of a chiral sulfamic acid of formula (I):

$$(R_1, R_2)N\text{-}SO_3H \qquad (I)$$

wherein:

$R_1$ and $R_2$, being the same or different, are selected from the group consisting of a hydrogen atom, a linear, branched or cyclic alkyl or heteroalkyl group, being substituted or not, or an aromatic or heteroaromatic group, being substituted or not, provided that at least one of the $R_1$ and $R_2$ groups is chiral.

Such compounds have a stronger acidity than corresponding carboxylic acids; on the basis whereof they will have an increased possibility of salt formation, particularly with weakly basic compounds.

In this respect, it is observed that cyclohexylsulfamic acid and it's sodium salt are known as sweeteners (having nr. E952).

The use of the present sulfamic acids as resolving agents is nevertheless not disclosed before.

It is observed that resolution of racemic compounds through formation and separation of diastereomeric salts remains an important technology for the preparation of enantiopure products on an industrial scale. The finding of a suitable resolving agent is however, often, trial and error. To overcome this problem, the present resolving agents provide a significant increased diversity, in that the groups $R_1$ and $R_2$ can basically represent any aliphatic or aromatic group, or one of them being hydrogen.

The mixture to be resolved according to the invention is preferably a basic mixture.

In the first aspect of the invention, the resolving agent is a chiral sulfamic acid, wherein the aliphatic group, represented by $R_1$ and/or $R_2$ is a hydrogen atom or $R_1$ and/or $R_2$ is a $C_1$-$C_{30}$ linear, branched and/or cyclic alkyl group being substituted or not, or wherein $R_1$ and/or $R_2$ represent an aromatic group or heteroaromatic group; having from 3 to 30 C-atoms, said hetero atom being selected from N, P, O and S, provided that at least one of the $R_1$ and $R_2$ groups is chiral.

It is nevertheless observed that the above enumerations are not limitative; $R_1$ and $R_2$ can represent other groups, provided that the sulfamic acid is still chiral.

With respect to the present resolving agents reference is made to Stereochem. Org. Compounds, E.L. Eliel, S.H. Wilen, Wiley Interscience, N.Y. 1994, pp. 322-337, mentioning the use of 10-camphorsulfonic acid as a resolving agent for bases. Any suggestion about the use of sulfamic acids as resolving agents is nevertheless missing in this reference.

The invention further relates to a process for the preparation of a chiral sulfamic acid wherein an amine of formula (II) :

$$(R_1, R_2)N\text{-}H \qquad (II)$$

wherein $R_1$ and $R_2$ are as defined above, is reacted with a suitable reagent to obtain said chiral sulfamic acid.

In principle, all commercially available chiral amines can be used in the above process, for example, (R)-1-(4-nitrophenyl)ethylamine hydrochloride, (+)-dehydroabiethylamine, (S)-2-amino-1,1-diphenyl propanol, D-phenylalaninol, L(-)-α-amino-ε-caprolactam, (R)-(-)-1-amino-2-propanol, (R)-(+)-1-(1-naphthyl)ethylamine, (R)-(+)-1-(2-naphthyl)ethylamine, (R)-(+)-1-(4-bromophenyl)ethylamine, (R)-(+)-α-methylbenzylamine, (S)-(-)-1-(1-naphthyl)ethylamine, (S)-(-)-1-(2-naphthyl)ethylamine, (S)-(-)-1-(4-bromophenyl)ethylamine, (S)-(-)-α-methylbenzylamine, (S)-(+)-1-amino-2-propanol, (R)-(+)-bornylamine, (R)-(-)-phenylglycinol, (S)-(+)-phenylglycinol, D-(+)-phenylalaninol, L-(-)-phenylalaninol, (1S,2S)-(+)-2-amino-3-methoxy-1-phenyl-1-propanol, (-)-cis-myrtanylamine, D-(+)-norephedrine, L-(-)-norephedrine, (1R,2R)-(-)-2-amino-1-phenyl-1,3-propanediol, (1S,2S)-(+)-2-amino-1-phenyl-1,3-propanediol, (R)-(-)-1-aminoindan, (S)-(+)-1-aminoindan, (-)-isopinocamphenylamine, (+)-isopinocamphenylamine, (1R,2S)-(-)-cis-1-amino-2-indanol, (1S,2R)-(+)-cis-1-amino-2-indanol, (S)-2-phenylglycine methyl ester hydrochloride, (S)-2-phenylglycine methyl ester hydrochloride, (-)-L-phenylalanine benzyl ester, (R)-1-(3-methoxyphenyl)ethylamine, (S)-1-(3-methoxyphenyl)ethylamine, (R)-1-(4-methoxyphenyl)ethylamine, (S)-1-(4-methoxyphenyl)ethylamine, (1R,2S)-(+)-cis-[2-(benzylamino)cyclohexyl]methanol, (-)-bis[(S)-1-phenylethyl]amine hydrochloride, (-)-ephedrine, (+)-bis-[(R)-1-phenylethyl]amine hydrochloride, (+)-ephedrine hydrochloride, (1S,2R)-(-)-cis-[2-(benzylamino)cyclohexyl]methanol, (R)-benzyl-1-(1-naphthyl)ethylamine hydrochloride, (S)-1-(4-nitrophenyl)ethylamine hydrochloride, (S)-benzyl-1-(1-naphthyl)ethylamine hydrochloride, (R)-(+)-N-benzyl-1-phenylethylamine, (R)-(+)-N-methyl-1-phenylethylamine, (1R,2S)-(-)-N-methylephedrine, brucine, quinine, (-)-strychnine, cinchonidine, cichonine, quinidine, (R)-(+)-N,N-dimethyl-1-phenylethylamine, (S)-(-)-N,N-dimethyl-1-phenylethylamine, D-arginine, D-aspartic acid, D-glutamic acid, D-valine, L-aspartic acid, L-glutamic acid, L-valine.

Because many starting amines are inexpensive reagents, and their conversion to sulfamic acids utilizes inexpensive reagents, this invention provides the basis for the large-scale use of sulfamic acids in diastereomer mediated resolutions.

Preferably, the suitable reagent to be used in the above process, is chlorosulfonic acid, sulfur trioxide, sulfur trioxide pyridine complex, sulfur trioxide dimethylformamide complex or any other adduct of sulfur trioxide.

The majority of commercially available acidic resolving agents are carboxylic acids (although there are a small number of more acidic sulfonic acid systems available); consequently there is a lack of diversity when one requires resolution of a weakly basic system. Since sulfamic acids are more acidic than carboxylic acids, they will be advantageous in the resolution of weakly basic systems.The current method provides a significant enhancement in diversity.

The invention therefore relates to a process of resolving a racemic base (B) of formula (III); by reacting said base (B) with homochiral sulfamic acid of formula (I):

$$(R_1, R_2)N\text{-}SO_3H \qquad (I)$$

wherein $R_1$ and $R_2$ are as defined before, to obtain the diastereomeric salts of formula (IV) :

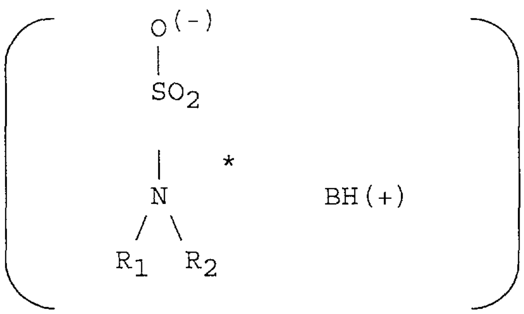

(IV)

whereof either the p- or the n-diastereomeric salt will crystallize, which salt crystals are recovered and thereafter treated with a base to obtain the base (B) in an optically enriched state and said enantiomerically pure, chiral sulfamic acid of formula (I):

$$(R_1, R_2)N\text{-}SO_3H \qquad (I)$$

which can be recycled, if desired.

This process is schematically represented as follows:

Scheme 1:

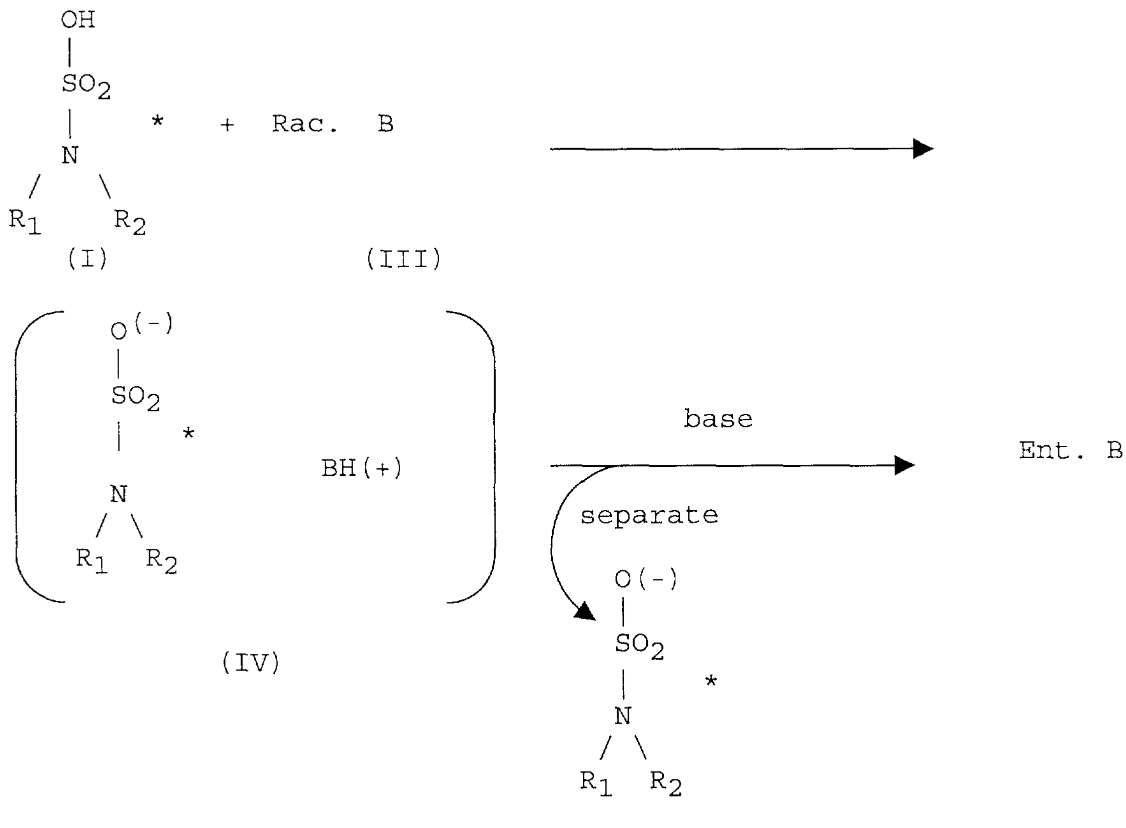


(I) (III) (IV) (I)

In the second aspect of the invention, racemic sulfamic acids (which may be prepared from racemic amines) can on the other hand also be used as starting materials to be resolved. The invention therefore relates in another aspect to a process of resolving a racemic sulfamic acid of formula (I):

$$(R_1, R_2)N\text{-}SO_3H \qquad (I)$$

wherein $R_1$ and $R_2$ are as defined above, by reacting said racemic sulfamic acid with an enantiomerically pure basic resolving agent (A) to obtain the diastereomeric salt of formula (V)

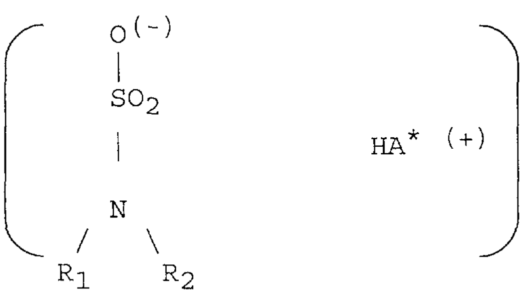

(V)

whereof either the p- or n-salt will crystallize, which salt crystals are recovered, and thereafter treated with an acid to

obtain the optically pure sulfamic acid of formula (I), and said resolving agent (A), which can be recycled, if desired.

In a third and closely related aspect, it is apparent that using the approach described above that it is possible to use a given chiral amine to resolve a racemic amine of the same structural formula *via* intermediacy of sulfamate formation. In a preferred embodiment of the invention special, and most advantageous circumstances apply when i) the salt which crystallizes where the chirality of the R groups about the separate nitrogen atoms have the same absolute stereochemistry respectively, and ii) that the resolving agent may be added in substoichiometric amounts (most preferably 0.5 eq.).

Case A: The compound to be resolved is a racemic sulfamic acid of formula (I):

$$Rac\text{-}(R_1, R_2)N\text{-}SO_3H \qquad (I)$$

wherein $R_1$ and $R_2$ are as defined above, and may be obtained by reaction of a racemic amine of formula (II):

$$(R_1, R_2)NH \qquad (II)$$

with a suitable reagent to form the racemic sulfonic acid (I). Thereafter salt formation with a substoichiometric quantity of chiral amine (II) may give rise to a diastereomeric salt where the respective $R_1$ and $R_2$ groups about each nitrogen have the same absolute stereochemistry respectively, and therefore mother liquors contain the free amine, which is antipodal with respect to the $R_1$ and $R_2$ groups, in an enantioenriched form.

Case B: The racemate to be resolved may be a racemic amine of formula (II) :

$$Rac\text{-}(R_1, R_2)NH \qquad (II)$$

Salt formation with a substoichiometric quantity of homochiral sulfamic acid (I) may give rise to a diastereomeric salt where the respective R groups about each nitrogen have the same absolute stereochemistry respectively, and therefore mother liquors contain the free amine, which is antipodal with respect R groups, in an enantioenriched form.

$$Ent\text{-}(R_1, R_2)N\text{-}SO_3H \qquad (I)$$

The above-mentioned processes can schematically be represented as follows:

## Case A:

**step a:**

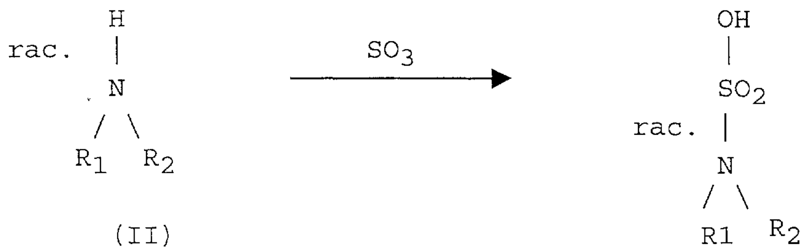


(II)

(I)

**step b:**

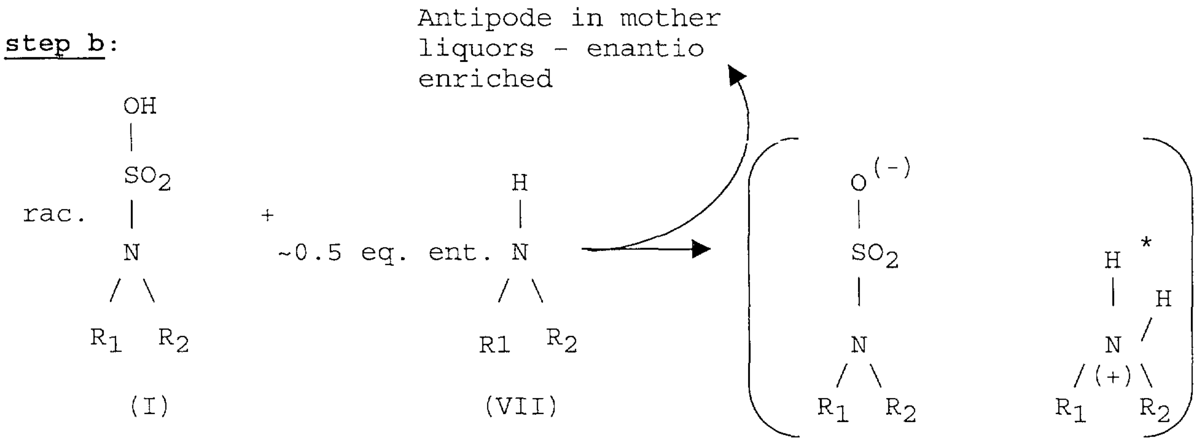


(I)

(VII)

(VI) - cryst.

**step c:**

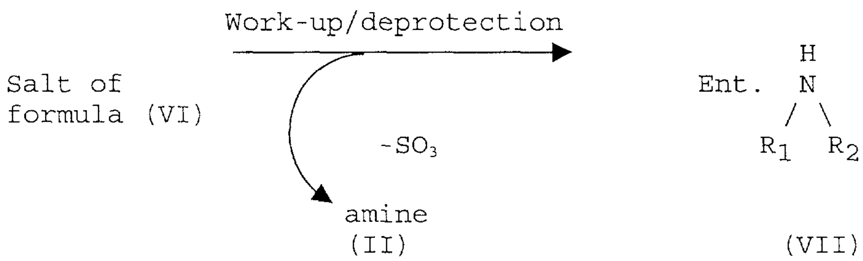


(VII)

**Case B:**

**step a:**

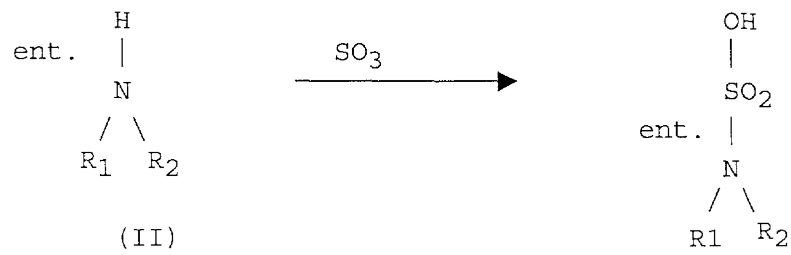


(II) (I)

**step b:**

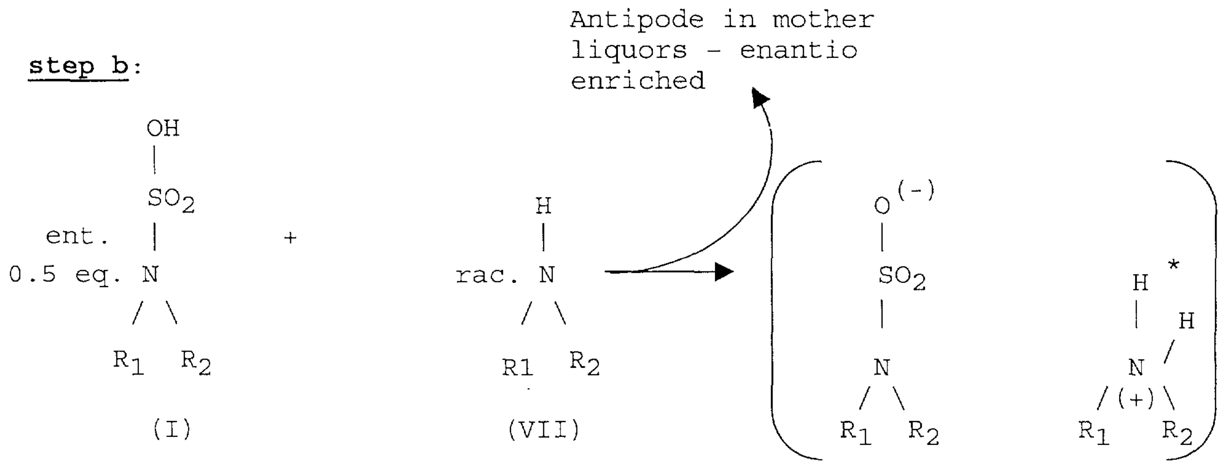


(I) (VII) (IX) - cryst.

**step c:**

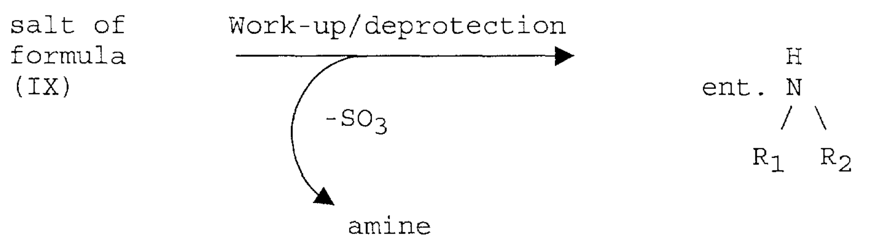


(VII)

More specifically, in step (b, Case A & B) the salt crystallizes, which crystals are collected and treated in step (c) with a reagent, such as aqueous hydrochloric resulting in in *situ* removal of the $SO_3$ group providing the enantioenriched amine of formula (II). According to a very preferred embodiment, the molar ratio of the resolving agent (VII in Case A, I in Case B) to said racemate is about 0.5 eq. The resolving agent is regenerated as the product. A portion of the product may be used in a subsequent resolution and since the antipode is isolated in an enantioenriched form in the mother liquor stream, this aspect of the invention provides the basis of a steady state self-resolution process.

This use of substoichiometric amounts of resolving agent is precedented in diastereomer mediated resolutions and is known as the method of Pope and Peachey or the method of half quantities, and is disclosed in Enantiomers, Racemates and Resolutions, Wiley and Sons, New York (1981), pp. 309-313.

Example 1

Preparation of D-phenylalaninol sulfamic acid

500 mg (3.31 mmol) D-phenylalaninol was dissolved in 10 ml of demi-water. The pH of the mixture was adjusted to pH 9.5-10.0 via addition of an aqueous 1.0 N NaOH solution. 659 mg (3.64 mmol)of sulfur trioxide pyridine complex was added in small portions over a period of 0.5 h with constant addition of 1.0 N NaOH solution to maintain a pH of 9.5-10.0. After the reaction was complete the reaction mixture was concentrated to remove the pyridine. Ethanol was added and the formed precipitate was removed by filtration and discarded. Acetone was added and the product precipitated from the solution. Filtration yielded 537 mg (2.32 mmol) of the sodium salt of D-phenylalaninol sulfamic acid (70 %).
Next, 92 mg (0.36 mmol) of the sodium salt of D-phenylalaninol sulfamic acid was dissolved in 5 ml demineralized water. 3 ml of a suspension of Dowex-$H^+$ in demineralized water was added and stirred for 5 min. The mixture was filtered, the resin was rinsed 4 times with 5 ml of demineralized water and the collected aqueous filtrate was freeze-dried. 85 mg (0.36 mmol) of D-phenylalaninol sulfamic acid was obtained after freeze-drying.

Example 2

Resolution of racemic $\alpha$-methylbenzylamine

80 mg (0.34 mmol) of D-phenylalaninol sulfamic acid was dissolved in 15 ml ethanol after which 43 mg (0.35 mmol) of racemic $\alpha$-methylbenzylamine dissolved in 2 ml ethanol was added. The solvent was evaporated to a total volume of 3 ml and cooled down to accelerate crystallisation. After crystallisation both mother liquor and crystals were analysed by HPLC on a chiral ODH-column (eluent hexane: isopropylalcohol 90:10) to determine the relative amount of (S)- and (R)-$\alpha$-methylbenzylamine. HPLC analysis showed enantiomeric enrichment of both the crystals and the mother liquor.

Example 3

Preparation of (D)-1-(-3-methoxyphenyl)ethyl sulfamic acid and resolution of racemic $\alpha$-methylbenzylamine

1.17 g (7.73 mmol) (D)-1-(-3-methoxyphenyl)ethyl amine was added dropwise to a suspension of 1.32 g (8.63 mmol) sulfur trioxide N,N-dimethylformamide complex in 15 ml of THF. After stirring for 10 min. TLC-analysis showed complete disappearance of primary amine (ninhydrine colouring). After evaporation of the solvents the sulfamic acid was obtained as a clear oil.
87 mg (0.38 mmol) of the sulfamic acid of (D)-1-(-3-methoxyphenyl)ethyl amine was dissolved in 1 ml of acetone and 48 mg (0.40 mmol) of racemic $\alpha$-methylbenzylamine in 1 ml acetone was added. Within an hour crystals were formed and HPLC analysis showed enantiomeric enrichment of both the crystals and the mother liquor.

## Claims

**1.** A resolving agent for resolving a mixture of enantiomers, the resolving agent consisting of a chiral sulfamic acid of formula (I):

$$(R_1, R_2)N\text{-}SO_3H \qquad (I)$$

wherein:

$R_1$ and $R_2$, being the same or different, are selected from the group consisting of a hydrogen atom, a linear, branched or cyclic alkyl or heteroalkyl group, being substituted or not, or an aromatic or heteroaromatic group, being substituted or not, provided that at least one of the $R_1$ and $R_2$ groups is chiral.

**2.** A resolving agent according to claim 1, wherein said mixture of enantiomers is a basic mixture.

**3.** A resolving agent according to claim 1 or 2, wherein said alkyl group is a $C_1$-$C_{30}$ alkyl group, said aromatic group

is a group having from 3 to 30 C-atoms, and said hetero atom is selected from N, P, O and S.

**4.** A process for the preparation of a chiral sulfamic acid according to claim 1, from an amine of formula (II) :

$$(R_1, R_2)N\text{-}H \qquad \text{(II)}$$

wherein $R_1$ and $R_2$ are as defined in claim 1, by reaction of said amine with a suitable reagent to obtain said chiral sulfamic acid of formula (I).

**5.** A process according to claim 4, wherein said reagent is chlorosulfonic acid, sulfur trioxide, sulfur trioxide pyridine complex, sulfur trioxide dimethylformamide complex, or other adducts of sulfur trioxide.

**6.** A process according to claim 4 to 5, of resolving a basic mixture of enantiomers (B) by reacting said basic mixture with a homochiral sulfamic acid of formula (I):

$$(R_1, R_2)N\text{-}SO_3H \qquad \text{(I)}$$

wherein $R_1$ and $R_2$ are as defined in claim 1, to obtain the diastereomeric salts of formula (IV) :

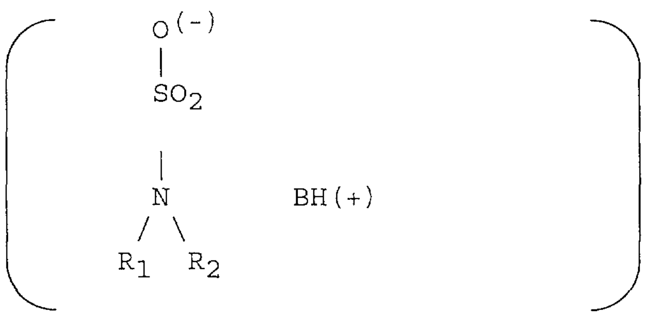

(IV)

whereof either the p- or the n-diastereomeric salt will crystallize, which salt crystals are recovered and thereafter treated with a base to obtain the base (B) in an optically enriched state, and said enantiomerically pure, chiral sulfamic acid of formula (I)

$$(R_1, R_2)N\text{-}SO_3H \qquad \text{(I)}$$

which can be recycled, if desired.

**7.** A process according to claim 4 to 6, of resolving a mixture of enantiomers of sulfamic acid of formula (I):

$$(R_1, R_2)N\text{-}SO_3H \qquad \text{(I)}$$

wherein $R_1$ and $R_2$ are as defined in claims 1 and 3, by reacting said sulfamic acid with an enantiomerically pure, basic resolving agent A to obtain the diastereomeric salt of formula (V)

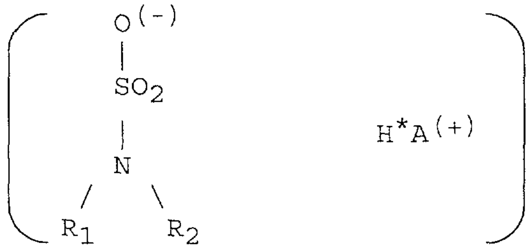

(V)

whereof either the p- or the n-diastereomeric salt will crystallize, which salt crystals are recovered, and thereafter treated with an acid to obtain the optically pure sulfamic acid of formula (I), and said resolving agent A, which can be recycled, if desired.

**8.** A process according to the claims 4 to 7, wherein the molar ratio of racemate to resolving agent is used in a substoichiometric amount and most preferably at 0.5 eq.

**9.** A process according to any of the claims 4 to 8, wherein the resolving agent is recycled.

**10.** A process according to claims 4 to 9, wherein the amine of formula (II):

$$(R_1, R_2)N\text{-}H \qquad (II)$$

is recovered from the corresponding sulfamic acid by removal of $SO_3$, preferably by treatment of said sulfamic acid with chemical or thermal means, and most preferably by treatment with a hydrohalogenic acid.

European Patent Office

## PARTIAL EUROPEAN SEARCH REPORT

which under Rule 45 of the European Patent Convention shall be considered, for the purposes of subsequent proceedings, as the European search report

Application Number EP 01 20 2251

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2 933 513 A (G. NOMINE, ET AL.) 19 April 1960 (1960-04-19) * claim 10 * | 1,3 | C07B57/00 C07C307/02 |
| X | A.G. LLOYD, ET AL.: "Preparation of 2-deoxy-2-'35S!sulphoamino-D-glucose" BIOCHEMICAL JOURNAL, vol. 92, 1964, pages 68-72, XP001030872 Portland Press, London, GB ISSN: 0264-6021 * the whole document * | 1,3 | |
| X | S.-K. CHUNG, ET AL.: "Synthesis and bioactivities of steroid derivatives as antifungal agents " TETRAHEDRON, vol. 54, no. 52, 24 December 1998 (1998-12-24), pages 15899-15914, XP004144536 Elsevier Science Publishers, Amsterdam, NL ISSN: 0040-4020 * compound 24 * | 1,3 | |
| | -/-- | | TECHNICAL FIELDS SEARCHED (Int.Cl.7) C07B C07C |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 8 November 2001 | English, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 01 20 2251

Claim(s) searched completely:
6-10

Claim(s) searched incompletely:
1-5

Reason for the limitation of the search:

The initial phase of the search revealed a very large number of documents relevant to the issue of novelty. So many documents were retrieved that it is impossible to determine which part(s) of the claim(s) may be said to define subject-matter for which protection might legitimately be sought (Article 84 EPC).

For these reasons it appears impossible to execute a meaningful search and /or to issue a complete search report over the whole breadth of the claim(s). The search and the report for those claims can only be considered complete for the compound of example 1

European Patent Office

# PARTIAL EUROPEAN SEARCH REPORT

Application Number
EP 01 20 2251

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | M.L. WOLFRAM, ET AL.: "Derivatives of D-glucose containing the sulphamino group" JOURNAL OF THE AMERICAN CHEMICAL SOCIETY, vol. 79, no. 18, 20 September 1957 (1957-09-20), pages 5043-5046, XP002182277 American Chemical Society, Washington, DC, US ISSN: 0002-7863 * compounds VI, IX, XI-XII * --- | 1,3 | |
| X | M.H. PAYNE, ET AL.: "Positional effects of sulphation in hirudin and hirudin PA related anticoaguant peptides" JOURNAL OF MEDICINAL CHEMISTRY, vol. 34, no. 3, March 1991 (1991-03), pages 1184-1187, XP002182278 American Chemical Society, Washington, DC, US ISSN: 0022-2623 * compounds 7, 12, 17; page 1187, lines 3-7 * --- | 1,3 | TECHNICAL FIELDS SEARCHED (Int.Cl.7) |
| X | R. COSSTICK, ET AL.: "Synthesis of some analogues of nucleoside 5'-triphosphates" TETRAHEDRON, vol. 40, no. 2, 1984, pages 427-431, XP002182279 Elsevier Science Publishers, Amsterdam, NL ISSN: 0040-4020 * compound 8 * --- -/-- | 1,3 | |

EPO FORM 1503 03.82 (P04C10)

European Patent Office

# PARTIAL EUROPEAN SEARCH REPORT

Application Number
EP 01 20 2251

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | I. MARLE, ET AL.: "Separation of enantiomers using cellulase (CBH I) silica as a chiral stationary phase" JOURNAL OF CHROMATOGRAPHY., vol. 586, no. 2, 22 November 1991 (1991-11-22), pages 233-248, XP002182280 Elsevier Science Publishers, Amsterdam, NL ISSN: 0021-9673 * page 235, left-hand column, line 19 - line 21 * --- | 1 | |
| X | B.M. KIM, ET AL.: "Efficient hydrolysis of beta-aminosulfamic acids using a Lewis acid and a thiol for the synthesis of 2,3-diaminopropanoate derivatives" TETRAHEDRON LETTERS, vol. 39, no. 30, 23 July 1998 (1998-07-23), pages 5381-5384, XP004123239 Elsevier Science Publishers, Amsterdam, NL ISSN: 0040-4039 * compound 2f * --- | 1 | TECHNICAL FIELDS SEARCHED (Int.Cl.7) |
| X | S.B. COHEN, ET AL.: "Synthesis of S-linked glycosyl amino acids in aqueous solution with unprotected carbohydrates" ORGANIC LETTERS, vol. 3, no. 3, 8 February 2001 (2001-02-08), pages 405-407, XP002182281 American Chemical Society, Washington, DC, US ISSN: 1523-7060 * compound 7a * --- -/-- | 1 | |

EPO FORM 1503 03.82 (P04C10)

European Patent Office

# PARTIAL EUROPEAN SEARCH REPORT

Application Number
EP 01 20 2251

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | J.E. BALDWIN, ET AL.: "Cyclic sulphamidates: new synthetic precursors for beta-functionalised alpha-amino acids" TETRAHEDRON: ASYMMETRY, vol. 1, no. 12, 1990, pages 881-884, XP002182282 Elsevier Science Publishers, Amsterdam, NL ISSN: 0957-4166 * compounds 6, 8 * --- | 1 | |
| A | US 4 582 928 A (S. AOKI, ET AL.) 15 April 1986 (1986-04-15) * claims 1,2; examples 6,7 * --- | 6-10 | |
| A | K.B. WIBERG: "Organic Syntheses, vol. 49" 1969 , JOHN WILEY AND SONS , NEW YORK, NY, US XP002182283 * page 93 - page 98 * --- | 6-10 | TECHNICAL FIELDS SEARCHED (Int.Cl.7) |
| A | A.H. BLATT: "Organic Syntheses, Collective Volume 2" 1946 , JOHN WILEY AND SONS , NEW YORK, NY, US XP002182284 * page 506 - page 509 * ----- | 6-10 | |

EPO FORM 1503 03.82 (P04C10)

## ANNEX TO THE EUROPEAN SEARCH REPORT ON EUROPEAN PATENT APPLICATION NO. EP 01 20 2251

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2001

| Patent document cited in search report | | Publication date | Patent family member(s) | | | Publication date |
|---|---|---|---|---|---|---|
| US 2933513 | A | 19-04-1960 | NONE | | | |
| US 4582928 | A | 15-04-1986 | JP | 1582059 | C | 11-10-1990 |
| | | | JP | 2007585 | B | 19-02-1990 |
| | | | JP | 60252454 | A | 13-12-1985 |
| | | | DE | 3565530 | D1 | 17-11-1988 |
| | | | EP | 0158301 | A2 | 16-10-1985 |
| | | | ES | 541857 | D0 | 16-05-1986 |
| | | | ES | 8607215 | A1 | 01-11-1986 |

EPO FORM P0459

For more details about this annex : see Official Journal of the European Patent Office, No. 12/82